Europäisches Patentamt

European Patent Office

Office européen des brevets

(11) Publication number: **0 204 459**
**A2**

## EUROPEAN PATENT APPLICATION

(21) Application number: **86303789.1**

(22) Date of filing: **19.05.86**

(51) Int. Cl.⁴: **A 61 B 5/02**
**G 01 N 21/31**

(30) Priority: **06.06.85 US 741937**

(43) Date of publication of application:
**10.12.86 Bulletin 86/50**

(84) Designated Contracting States:
**DE FR GB NL**

(71) Applicant: **The BOC Group, Inc.**
**85 Chestnut Ridge Road**
**Montvale, New Jersey 07645(US)**

(72) Inventor: **Haisley, Charles Keith**
**2675 Stephens Road**
**Boulder Colorado 80303(US)**

(74) Representative: **Gough, Peter et al,**
**c/o THE BOC GROUP PLC Chertsey Road**
**Windlesham Surrey GU20 6HJ(GB)**

(54) Oximeter finger probe.

(57) A finger probe 10 for use with an oximeter includes an upper housing 20 and a lower housing 22 pivotably hinged together at their distal ends 12 such that a human finger can be inserted into the proximal end 14 of the finger probe into a chamber 26 formed therebetween. The probe 10 contains at least one light emitter 50 and at least one light detector 38 situated within the upper and lower housings so as to allow light from the emitter 50 to pass through the finger to the detector 38. At least one emitter 50 is contained within an emitter housing 42 that is pivotably mounted to the upper housing 20 such that it conforms to the shape of the patient's finger when inserted into the chamber 26. Also, wiring 16 for the emitter 50 and detector 38 depends outwardly from the proximal end 14 of the finger probe along the finger such that the wiring can be readily taped to the finger and, thus, help retain the finger probe 10 in position on the finger.

**FIG. 2**

## TITLE

### OXIMETER FINGER PROBE

The present invention relates to a finger probe, for use particularly with an oximeter, which surrounds the end of a patient's finger. Such finger probes are utilized to contain a light emitter and light detector and to position the same such that the light passes through the finger as it goes from the emitter to the detector. Similar finger probes are used for pulse detectors, which may include probes that reflect the light from arterial passages such that the emitter and detector are on the same side of the finger.

Typically, the finger probes are intended to be easily attached and detached from the end of the patient's finger, yet when installed, they minimize the amount of stray light entering the probe where such light might interfere with the emission and/or detection of the light that is used to carry out the particular measurement.

In oximeters, typical of which is described in U.S. Patent 4,407,290, two emitters are used, one emitting light in the red wavelength range and the other acting in the infrared range. As described in the aforesaid patent, only one detector need be provided as only one emitter is operational at any one period of time.

Aside from convenience and shielding, therefore, it is important that the emitter conform, to the extent possible, to the finger so that the emitter can rest directly upon the finger.

Obviously, due to the variety of sizes and shapes of fingers, the finger probe, therefore, needs be built with some flexibility or adjustment without contributing greatly to the cost or manufacturability of the device.

In addition, a desired feature is to provide wiring to the emitter and detector that is not in the way of the unit and preferably can even be used to stabilize the finger probe when attached to the patient's finger.

According to the present invention a finger probe for positioning on the end of a patient's finger is characterised by an upper housing and a lower housing, said upper housing and said lower housing being pivotably connected together at the distal ends thereof, said upper housing and said lower housing thereby forming an internal chamber open at the proximal end for receiving the finger, light detector means affixed to one housing, and a light emitter housing pivotably connected to the other housing, said light emitter housing having a light emitter adapted to direct light across said internal chamber to be received by said light detector means, said pivotal connection of said light emitter housing allowing movement thereof to conform to the profile of a finger positioned in said internal chamber.

In a preferred embodiment the finger probe utilizes a pair of housings, an upper housing and a lower housing which are pivotably hinged together at the distal end of the probe. Thus, by using a spring bias, the finger probe can be opened and closed to allow the finger to be inserted into the proximal end and the finger probe retained thereon. The upper and lower housings are of solid, molded construction and, when closed, overlap to seal out undesired light.

At least one emitter and at least one detector are contained within the finger probe such that they are fixed to direct the light through the finger inserted in the probe.

Preferably, the light emitter is contained in an emitter housing that is pivotably mounted to one of the upper or lower housings, preferably the upper housing. The point about which the emitter housing pivots is located such that it conforms to the profile of a finger inserted into the finger probe and seats comfortably and firmly thereagainst. Thus, the finger probe has the advantage of ease of attachment and detachment from a finger, being pivoted at one end, and yet the emitter itself lends a certain flexibility to the probe by being movable with respect to the housing to which it is affixed to conform to the wide variety of finger profiles.

Additionally, the wiring that connects the electronic instrument to the light emitter and detector depends outwardly from the proximal end of the finger probe, that is, the end of the finger probe into which the finger is inserted. The wiring, thus, may include a cable strain relief adjacent to its entry into the finger probe and which follows the general contour of the finger itself.

Therefore, it is relatively convenient, when necessary to more firmly attach the finger probe to the patient's finger, to tape that wire or cable strain relief means directly to the finger, thus, assuring against inadvertent removal of the finger probe.

An embodiment of the invention will now be described, by way of example, reference being made to the Figures of the accompanying diagrammatic drawings in which :-

FIG. 1 is a perspective view showing a finger probe of the present invention installed on the end of a patient's finger;

FIG. 2 is a side cross-sectional view of the finger probe of FIG. 1;

FIG. 3 is an end view of the finger probe of FIG. 1; and

FIG. 4 is an exploded view showing the various parts of the finger probe of FIG. 1.

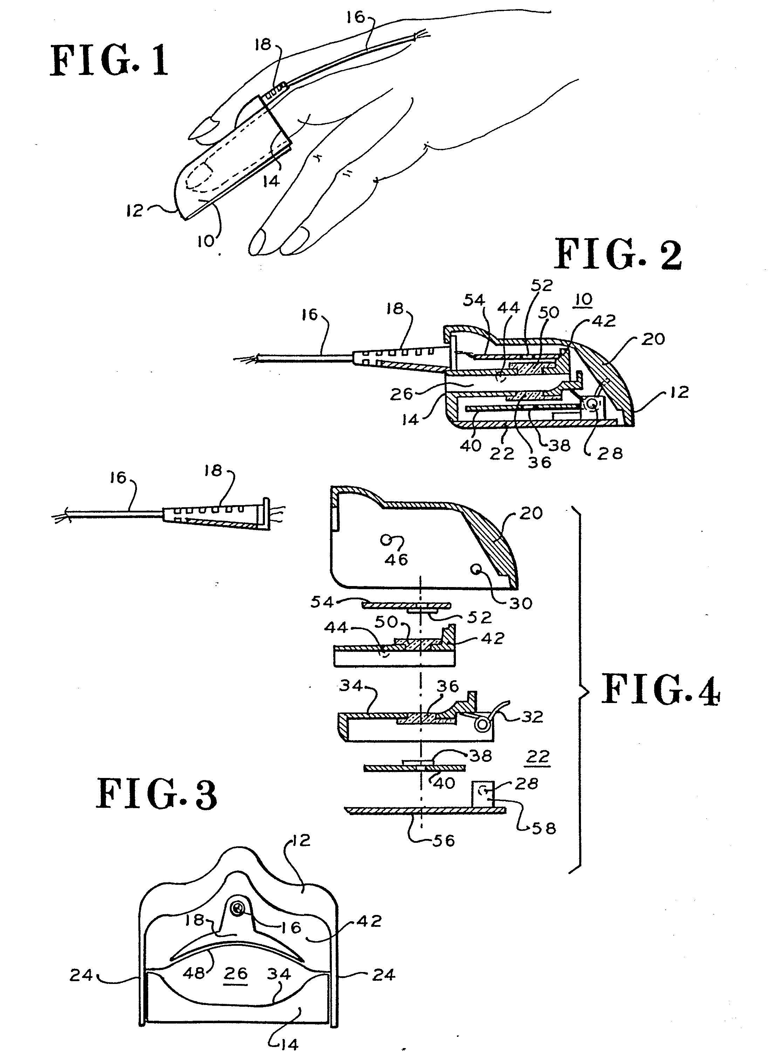

In FIG. 1 there is shown a perspective view of a finger probe 10 attached to the end of a patient's finger. As will be consistent in the following specification, the distal end 12 of finger probe 10 is defined as that end furthest out on the patient's finger and the proximal end 14 of finger probe 10 is the end into which the patient's finger is inserted. As is also noted in FIG. 1, the wires 16 for connecting the finger probe 10 to its associated electronic components extends outwardly from the proximal end 14 and generally conforms to the contour of the patient's finger. As part of the wires 16, a strain relief flap 18 is provided as the wires 16 enter the finger probe 10, the purpose of which is to both provide strain relief for the wires 16, but also to be a surface that is conveniently taped to the finger to insure that the finger probe 10 is retained in its position on the finger and inadvertent dislodgment is minimized.

Turning now to FIG. 2, there is shown a side cross-sectional view of the finger probe 10 and which comprises an upper housing 20 and a lower housing 22, each of which are preferably of polycarbonate material and molded into their desired shapes. The upper housing 20 includes sides 24 (see FIG. 3) that enclose the lower housing 22 and prevent the admission of undesired light into the internal chamber 26 that is formed in between upper housing 20 and lower housing 22 and where the patient's finger is positioned during use.

Upper housing 20 and lower housing 22 are pivotably connected together at the distal end 12 of finger probe 10 by pegs 28 which extend outwardly from the lower housing 22 and fit within recesses 30 (shown only in FIG. 4) formed on the inside surface of upper housing 20. Thus, the upper housing 20 and lower housing 22 can be moved with respect to each other about their pivotable connection, thus, opening or closing the opening in the proximal end 14. A spring bias, in the form of spring 32 is provided such that the spring bias urges upper housing 20 and lower housing 22 toward the closed position.

The lower housing 22 (taking again FIGS. 2 and 3) has, on its upper face, a curved surface 34 so as to conform to the general shape of a patient's finger. In the curved surface 34, there is positioned a lens 36, the purpose of which will become apparent. The lens 36 may be of optically clear acrylic material and may be held by a suitable adhesive in a corresponding opening in the curved surface 34. Positioned directly below the lens 36 is a light detector 38 that is mounted on a board 40 containing wiring and the like for light detector 38. The light detector 38 can preferably be mounted to board 40 by epoxy and board 40 contain the necessary wiring runs for connection between light detector 38 ultimately to wires 16. The actual wiring is not shown but is by conventional means.

Movably connected to the upper housing 20 is a light emitter housing 42. As shown is FIG. 2, the movable connection is accomplished by connecting light emitter housing 42 to the upper housing 20 by pegs 44 which depend outwardly from both sides of light emitter housing 42 and interfit within recesses 46 (one of which is shown in FIG. 4) in the internal surface of upper housing 20. In such manner, the light emitter housing 42 pivots about the pegs 44 and, thus, can move to conform to the particular profile of the finger on which finger probe 10 is positioned. Light emitter housing 42 has a curved surface 48 (FIG. 3 only) that faces the finger when in place, and into that curved surface is located a lens 50. Again, lens 50 may be made of an optically clear acrylic material adhesively fixed to curved surface 48.

Directly above lens 50 is a light emitter 52 which, when energized, emits light of a predetermined wavelength toward light detector 38. Obviously, when a finger is positioned within internal chamber 26, that light from light emitter 52 will pass directly through that finger and be received by light detector 38.

In the particular use to which this specific embodiment is intended, light emitter 52 comprises two LEDs, one transmitting light in the red wavelength range and the other transmitting light in the infrared range, the purpose of which may be more readily understood by reading the aforedescribed U.S. Patent 4,407,290.

The light emitter 52 shall be referred to generically hereinafter and is mounted such as by epoxy to a board 54 for electrical connections and containing appropriate conductive runs to ultimately be connected to wires 16 by conventional means.

It should be noted, however, that the movable connection between light emitter housing 42 and upper housing 20 permits the light emitter housing 42 to both conform to the particular finger profile and, thus, allow individual adaptation to various finger sizes and shapes to insure that light emitter 52 is best positioned adjacent to the finger for optimum transmission of light. Yet, the overall finger probe 10 is solidly constructed of two housings, each of molded material and joined together by a pivotable connection at the distal end 12.

Turning finally to FIG. 4, there is shown an exploded view of the various components of the assembly of finger probe 10.

The lower housing 22 is formed by a plurality of components including the curved surface 34 in which lens 36 is positioned, the board 40 for light detector 38 and a bottom 56 from which depends upwardly posts 58 from which pegs 28 depend outwardly and which fit within recesses 30 formed in the interior surface of upper housing 20 to create the pivotable connection therebetween.

The light emitter housing 42 which comprises its lens 50 and on which board 54 containing light emitter 52 is mounted. Depending outwardly from light emitter housing 42 are the pegs 44 and which fit within recesses 46 formed in the interior surface of upper housing 20, and this is movably mounted thereto by a pivotable connection such that the light emitter housing 42 conforms to a variety of finger profiles.

0204459

I CLAIM:

1. A finger probe 10 for positioning on the end of a patient's finger, said finger probe 10 <u>being characterised by</u> an upper housing 20 and a lower housing 22, said upper housing 20 and said lower housing 22 being pivotably connected together at the distal ends 12 thereof, said upper housing 20 and said lower housing 22 thereby forming an internal chamber 26 open at the proximal end 14 for receiving the finger, light detector means 38 affixed to one housing 22, and a light emitter housing 42 pivotably connected to the other housing 20, said light emitter housing 42 having a light emitter 52 adapted to direct light across said internal chamber 26 to be received by said light detector means 38, said pivotal connection of said light emitter housing 42 allowing movement thereof to conform to the profile of a finger positioned in said internal chamber 26.

2. A finger probe as claimed in Claim 1, <u>characterised in that</u> the light detector means 38 is affixed to the lower housing 22 whilst the light emitter housing 42 is pivotally connected to the upper housing 20.

3. A finger probe as claimed in Claim 1 or Claim 2 <u>characterised by</u> spring bias means 32 to bias the proximal ends 14 of said upper housing 20 and said lower housing 22 towards each other.

4. A finger probe as claimed in Claim 2 or Claim 3 <u>characterised in that</u> said lower housing 22 and said light emitter housing 42 each have curved surfaces 34, 48 facing the finger when positioned within said internal chamber.

PG/NJP/8609 EPC

5. A finger probe as claimed in anyone of Claims 1 to 4, <u>characterised in that</u> said light detector means 38 and said light emitter 52 include wiring means to wires external of said finger probe, said external wiring comprising wires 16 depending outwardly from said proximal end of said finger probe and generally conforming to the finger positioned within said finger probe.

6. A finger probe as claimed in Claim 5 <u>characterised in that</u> said external wiring 16 includes a strain relief flap 18 adjacent and depending outwardly from said light emitter housing 42, said strain relief flap 18 being shaped to conform to the finger position within said finger probe and adapted to be readily taped to the finger.

7. A finger probe as claimed in any one of Claims 2 to 6 <u>characterised in that</u> said pivotable connection between said light emitter housing 42 and said upper housing 20 comprises pegs 44 depending outwardly from said light emitter housing 42 and fitted within recesses 46 formed in the internal surface of said upper housing 20.

FIG. 1

FIG. 2

FIG. 4

FIG. 3

0204459

1/1